# EUROPEAN PATENT SPECIFICATION

(11) **EP 3 164 052 B1**
(45) Date of publication and mention of the grant of the patent: **02.09.2020**
(21) Application number: 15815581.2
(22) Date of filing: 30.06.2015
(51) Int. Cl.: A61B 1/267, A61B 6/03, A61B 1/00, A61B 6/00

(54) **SYSTEMS FOR MARKING BIOPSY LOCATION**
VORRICHTUNGEN ZUR MARKIERUNG EINER BIOPSIESTELLE
SYSTEMES DE MARQUAGE DE L'EMPLACEMENT D'UNE BIOPSIE

(30) Priority: 02.07.2014 US 201462020177 P; 29.06.2015 US 201514753229
(43) Date of publication of application: 10.05.2017
(73) Proprietor: Covidien LP, Mansfield, MA 02048 (US)
(72) Inventor: BROWN, Andrew E., St. Paul, Minnesota 55127 (US); LACHMANOVICH, Elad D., 7176682 Modin (IL); KLEIN, Eyal, Tel Aviv (IL); SHPINER, Tatyana, Nesher (IL); WEINGARTEN, Oren P., 45286 Hod Hasharon (IL)
(74) Representative: Maschio, Antonio
(86) International application number: PCT/US2015/038464
(87) International publication number: WO 2016/003990

(56) References cited:
- EP-A2- 2 253 287
- WO-A2-2008/111070
- US-A1- 2007 293 721
- US-A1- 2012 302 878
- US-A1- 2013 223 702
- US-A1- 2014 051 986

## Description

### BACKGROUND

### Technical Field

The present disclosure relates to biopsy location marking and to systems for marking the location of a biopsy on a bronchial tree model.

### Description of Related Art

A common device for inspecting the airway of a patient is a bronchoscope. Typically, the bronchoscope is inserted into a patient's airways through the patient's nose or mouth and can extend into the lungs of the patient. A typical bronchoscope includes an elongated flexible tube having an illumination assembly for illuminating the region distal to the bronchoscope's tip, an imaging assembly for providing a video image from the bronchoscope's tip, and a working channel through which instruments, e.g., diagnostic instruments such as biopsy tools, therapeutic instruments can be inserted.

Bronchoscopes, however, are limited in how far they may be advanced through the airways due to their size. Where the bronchoscope is too large to reach a target location deep in the lungs a clinician may utilize certain real-time imaging modalities such as fluoroscopy. Fluoroscopic images, while useful present certain drawbacks for navigation as it is often difficult to distinguish luminal passageways from solid tissue. Moreover, the images generated by the fluoroscope are two-dimensional whereas navigating the airways of a patient requires the ability to maneuver in three dimensions.

To address these issues systems have been developed that enable the development of three-dimensional models of the airways or other luminal networks, typically from a series of computed tomography (CT) images. One such system has been developed as part of the ILOGIC® ELECTROMAGNETIC NAVIGATION BRONCHOSCOPY® (ENB™), system currently sold by Covidien LP. The details of such a system are described in the commonly assigned U.S. Patent No. 7,233,820, filed on March 29, 2004 by Gilboa and entitled ENDOSCOPE STRUCTURES AND TECHNIQUES FOR NAVIGATING TO A TARGET IN BRANCHED STRUCTURE.

Document US2007/293721 discloses a system for marking a biopsy location according to the preamble of claim 1. While the system as described in U.S. Patent No. 7,233,820 is quite capable, there is always a need for development of improvements and additions to such systems.

### SUMMARY

The present invention is disclosed in the appended set of claims.

In an aspect of the present disclosure, the device of the invention is configured to carry out the step of loading a navigation plan into a navigation system with the navigation plan including a CT volume generated from a plurality of CT images, inserting a probe into a patient's airways with the probe including a location sensor in operative communication with the navigation system, registering a sensed location of the probe with the CT volume of the navigation plan, selecting a target in the navigation plan, navigating the probe and location sensor to the target, storing a position of the location sensor in the navigation system as a biopsy location, and performing a biopsy at the stored biopsy location.

In another aspect of the present disclosure, the device of the invention is configured to carry out the step of placing a virtual marker corresponding to the biopsy location in at least one of a 3D model of the patient's airways generated from the CT volume or a local view of the patient's airways generated from a slice of the CT volume.

In a further aspect of the present disclosure, it is disclosed a method, which is not part of the invention, which further includes locking the probe relative to the extended working channel.

In yet a further aspect of the present disclosure, the method, which is not part of the invention, further includes inserting the extended working channel and probe into a bronchoscope, and navigating them together to the target

In a further aspect of the present disclosure, the method, which is not part of the invention, further includes locking the extended working channel in position at the target when the location sensor is navigated to the target.

In yet a further aspect of the present disclosure, the method, which is not part of the invention, further includes removing the probe from the extended working channel and inserting a biopsy tool through the extended working channel to the target to perform the biopsy.

In another aspect of the present disclosure, the method, which is not part of the invention, further includes adjusting a position of the probe relative to the target, storing a second position of the location sensor in the navigation system as a second biopsy location, and performing a second biopsy at the second biopsy location.

In yet another aspect of the present disclosure, the method, which is not part of the invention, further includes selecting a second target in the navigation plan, navigating the probe and location sensor to the second target, storing a second position of the location sensor in the navigation system as a second biopsy location, and performing a biopsy at the stored second biopsy location.

In a further aspect of the present disclosure, the method, which is not part of the invention, further includes providing tissue from at least one of the biopsy location or the second biopsy location for rapid on-site evaluation.

In a further aspect of the present disclosure, the method, which is not part of the invention, further includes receiving results from the rapid on-site evaluation clinician indicating a need to return to at least one of the biopsy location or the second biopsy location, presenting a pathway to at least one of the biopsy location or the second biopsy location as a return target in the navigation plan based on the rapid on-site evaluation, navigating the location sensor to the return target, storing a return position of the location sensor in the navigation system as a return biopsy location, and performing at least one of an additional biopsy or a treatment at the stored return biopsy location.

In another aspect of the present disclosure, the method which is not part of the invention, further includes storing a distance to a center of the target and a biopsy position number with the biopsy location.

Any of the above aspects and embodiments of the present disclosure may be combined without departing from the scope of the present disclosure.

### BRIEF DESCRIPTION OF THE DRAWINGS

Objects and features of the presently disclosed system will become apparent to those of ordinary skill in the art when descriptions of various embodiments thereof are read with reference to the accompanying drawings, of which:
FIG. 1 is a perspective view of an electromagnetic navigation system in accordance with the present disclosure;
FIG. 2 is a schematic diagram of a workstation configured for use with the system of FIG. 1;
FIG. 3 is a flowchart illustrating a method for marking the location of a biopsy on a 3D model provided in accordance with the present disclosure, which is not part of the invention;
FIG. 4 is an illustration of a user interface of the workstation of FIG. 2 presenting a view for marking a biopsy location in accordance with the present disclosure;
FIG. 5 is an illustration of the user interface of the workstation of FIG. 2 presenting a view for marking a location of a biopsy or treatment of the target; and
FIG. 6 is an illustration of the user interface of the workstation of FIG. 2 presenting a view showing multiple marked biopsy locations.

### DETAILED DESCRIPTION

Systems for marking the location of a biopsy on a three-dimensional (3D) model are provided in accordance with the present disclosure and described in detail below. Various methods for generating the 3D model are envisioned, some of which are more fully described in co-pending U.S. Patent Application Nos. 13/838,805, 13/838,997, and 13/839,224, all entitled PATHWAY PLANNING SYSTEM AND METHOD, filed on March 15, 2013, by Baker. A location sensor may be incorporated into different types of tools and catheters to track the location and assist in navigation of the tools. Navigation of the location sensor or tool is more fully described in co-pending U.S. Provisional Patent Application No. 62/020,240, entitled SYSTEM AND METHOD FOR NAVIGATING WITHIN THE LUNG, filed on July 2, 2014, by Brown et al. The tracked location of the location sensor may also be used to virtually mark on a three-dimensional model of the airways of a patient the location within the airways of the patient where a biopsy or treatment is performed.

Additional features of the ENB system of the present disclosure are described in co-pending U.S. Provisional Patent Application Nos. 62/020,238, entitled INTELLIGENT DISPLAY, filed on July 2, 2014, by KEHAT et al.; 62/020,242, entitled UNIFIED COORDINATE SYSTEM FOR MULTIPLE CT SCANS OF PATIENT LUNGS, filed on July 2, 2014, by Greenburg; 62/020,245, entitled ALIGNMENT CT, filed on July 2, 2014, by Klein et al.; 62/020,250, entitled ALGORITHM FOR FLUOROSCOPIC POSE ESTIMATION, filed on July 2, 2014, by Merlet; 62/020,253, entitled TRACHEA MARKING, filed on July 2, 2014, by Lachmanovich et al.; 62/020,257, entitled AUTOMATIC DETECTION OF HUMAN LUNG TRACHEA, filed on July 2, 2014, by Markov et al.; 62/020,261, entitled LUNG AND PLEURA SEGMENTATION, filed on July 2, 2014, by Markov et al.; 62/020,258, entitled CONE VIEW - A METHOD OF PROVIDING DISTANCE AND ORIENTATION FEEDBACK WHILE NAVIGATING IN 3D, filed on July 2, 2014, by Lachmanovich et al.; and 62/020,262, entitled DYNAMIC 3D LUNG MAP VIEW FOR TOOL NAVIGATION INSIDE THE LUNG, filed on July 2, 2014, by Weingarten et al.

Detailed embodiments of such systems are described below. However, these detailed embodiments are merely examples of the disclosure, which may be embodied in various forms. Therefore, specific structural and functional details disclosed herein are not to be interpreted as limiting, but merely as a basis for the claims and as a representative basis for allowing one skilled in the art to variously employ the present disclosure in virtually any appropriately detailed structure. While the following embodiments are described in terms of bronchoscopy of a patient's airways, those skilled in the art will realize that the same or similar systems may be used in other lumen networks, such as, for example, the vascular, lymphatic, and/or gastrointestinal networks as well.

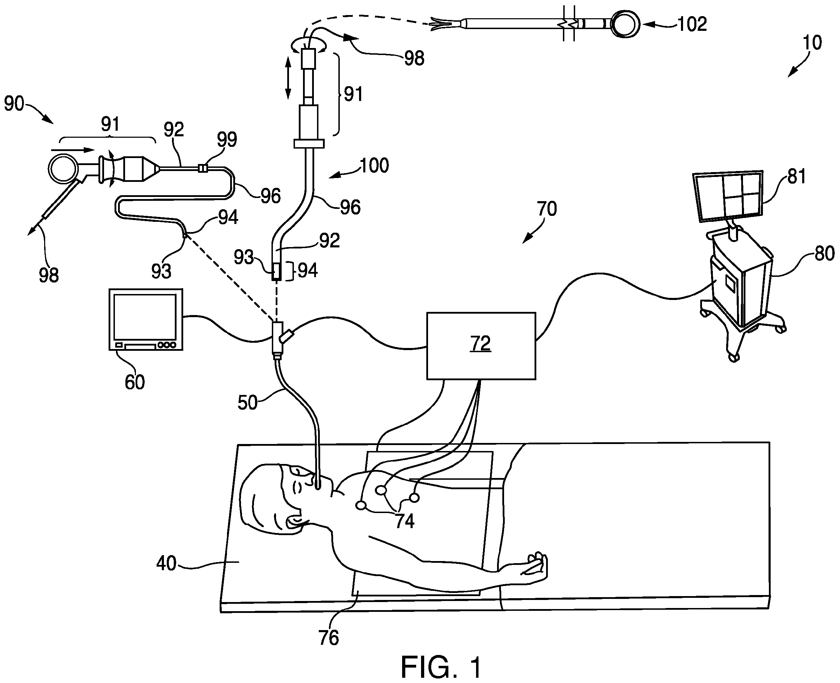

With reference to FIG. 1, an electromagnetic navigation (EMN) system 10 is provided in accordance with the present disclosure. One such ENM system is the ELECTROMAGNETIC NAVIGATION BRONCHOSCOPY® system currently sold by Covidien LP. Among other tasks that may be performed using the EMN system 10 are planning a pathway to target tissue, navigating a positioning assembly to the target tissue, navigating a biopsy tool to the target tissue to obtain a tissue sample from the target tissue using the biopsy tool digitally marking the location where the tissue sample was obtained, and placing one or more echogenic markers at or around the target.

EMN system 10 generally includes an operating table 40 configured to support a patient; a bronchoscope 50 configured for insertion through the patient's mouth and/or nose into the patient's airways; monitoring equipment 60 coupled to bronchoscope 50 for displaying video images received from bronchoscope 50; a tracking system 70 including a tracking module 72, a plurality of reference sensors 74, and an electromagnetic field generator 76; a workstation 80 including software and/or hardware used to facilitate pathway planning, identification of target tissue, navigation to target tissue, and digitally marking the biopsy location.

FIG. 1 also depicts two types of catheter guide assemblies 90, 100. Both catheter guide assemblies 90, 100 are usable with the EMN system 10 and share a number of common components. Each catheter guide assembly 90, 100 includes a handle 91, which is connected to an extended working channel (EWC) 96. The EWC 96 is sized for placement into the working channel of a bronchoscope 50. In operation, a locatable guide (LG) 92, including an electromagnetic (EM) sensor 94, is inserted into the EWC 96 and locked into position such that the sensor 94 extends a desired distance beyond the distal tip 93 of the EWC 96. The location of the EM sensor 94, and thus the distal end of the EWC 96, within an electromagnetic field generated by the electromagnetic field generator 76 can be derived by the tracking module 72, and the workstation 80. Catheter guide assemblies 90, 100 have different operating mechanisms, but each contain a handle 91 that can be manipulated by rotation and compression to steer the distal tip 93 of the LG 92, extended working channel 96. Catheter guide assemblies 90 are currently marketed and sold by Covidien LP under the name SUPERDIMENSION® Procedure Kits. Similarly catheter guide assemblies 100 are currently sold by Covidien LP under the name EDGE™ Procedure Kits. Both kits include a handle 91, extended working channel 96, and locatable guide 92. For a more detailed description of the catheter guide assemblies 90, 100 reference is made to commonly-owned U.S. Patent Application Serial No. 13/836,203 filed on March 15, 2013 by Ladtkow et al.

As illustrated in FIG. 1, the patient is shown lying on operating table 40 with bronchoscope 50 inserted through the patient's mouth and into the patient's airways. Bronchoscope 50 includes a source of illumination and a video imaging system (not explicitly shown) and is coupled to monitoring equipment 60, e.g., a video display, for displaying the video images received from the video imaging system of bronchoscope 50.

Catheter guide assemblies 90, 100 including LG 92 and EWC 96 are configured for insertion through a working channel of bronchoscope 50 into the patient's airways (although the catheter guide assemblies 90, 100 may alternatively be used without bronchoscope 50). The LG 92 and EWC 96 are selectively lockable relative to one another via a locking mechanism 99. A six degrees-of-freedom electromagnetic tracking system 70, e.g., similar to those disclosed in U.S. Patent No. 6,188,355 and published PCT Application Nos. WO 00/10456 and WO 01/67035, or any other suitable positioning measuring system is utilized for performing navigation, although other configurations are also contemplated. Tracking system 70 is configured for use with catheter guide assemblies 90, 100 to track the position of the EM sensor 94 as it moves in conjunction with the EWC 96 through the airways of the patient, as detailed below.

As shown in FIG. 1, electromagnetic field generator 76 is positioned beneath the patient. Electromagnetic field generator 76 and the plurality of reference sensors 74 are interconnected with tracking module 72, which derives the location of each reference sensor 74 in six degrees of freedom. One or more of reference sensors 74 are attached to the chest of the patient. The six degrees of freedom coordinates of reference sensors 74 are sent to workstation 80, which includes application 81 where sensors 74 are used to calculate a patient coordinate frame of reference.

Also shown in FIG. 1 is a catheter biopsy tool 102 that is insertable into the catheter guide assemblies 90, 100 following navigation to a target and removal of the LG 92. The biopsy tool 102 is used to collect one or more tissue sample from the target tissue. As detailed below, biopsy tool 102 is further configured for use in conjunction with tracking system 70 to facilitate navigation of biopsy tool 102 to the target tissue, tracking of a location of biopsy tool 102 as it is manipulated relative to the target tissue to obtain the tissue sample, and/or marking the location where the tissue sample was obtained.

Although navigation is detailed above with respect to EM sensor 94 being included in the LG 92 it is also envisioned that EM sensor 94 may be embedded or incorporated within biopsy tool 102 where biopsy tool 102 may alternatively be utilized for navigation without need of the LG or the necessary tool exchanges that use of the LG requires. A variety of useable biopsy tools are described in U.S. Provisional Patent Application Nos. 61/906,732 and 61/906,762 both entitled DEVICES, SYSTEMS, AND METHODS FOR NAVIGATING A BIOPSY TOOL TO A TARGET LOCATION AND OBTAINING A TISSUE SAMPLE USING THE SAME, filed November 20, 2013 and U.S. Provisional Patent Application No. 61/955,407 having the same title and filed March 14, 2014 and useable with the EMN system 10 as described herein.

During procedure planning, workstation 80 utilizes computed tomographic (CT) image data for generating and viewing a three-dimensional model ("3D model") of the patient's airways, enables the identification of target tissue on the 3D model (automatically, semi-automatically or manually), and allows for the selection of a pathway through the patient's airways to the target tissue. More specifically, the CT scans are processed and assembled into a 3D volume, which is then utilized to generate the 3D model of the patient's airways. The 3D model may be presented on a display monitor 81 associated with workstation 80, or in any other suitable fashion. Using workstation 80, various slices of the 3D volume and views of the 3D model may be presented and/or may be manipulated by a clinician to facilitate identification of a target and selection of a suitable pathway through the patient's airways to access the target. The 3D model may also show marks of the locations where previous biopsies were performed, including the dates, times, and other identifying information regarding the tissue samples obtained. These marks may also be selected as targets to which a pathway can be planned. Once selected, the pathway is saved for use during the navigation procedure. An example of a suitable pathway planning system and method is described in U.S. Patent Application Serial Nos. 13/838,805; 13/838,997; and 13/839,224, all entitled PATHWAY PLANNING SYSTEM AND METHOD, filed on March 15, 2014.

During navigation, EM sensor 94, in conjunction with tracking system 70, enables tracking of EM sensor 94 and/or biopsy tool 102 as EM sensor 94 or biopsy tool 102 is advanced through the patient's airways.

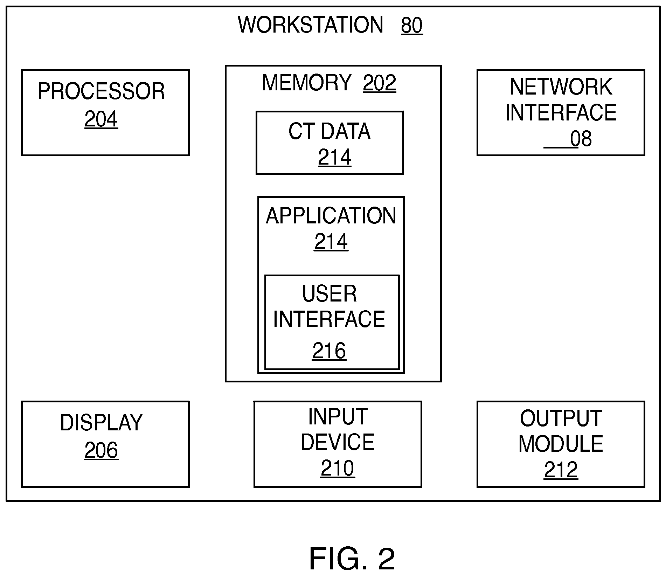

Turning now to FIG. 2, there is shown a system diagram of workstation 80. Workstation 80 may include memory 202, processor 204, display 206, network interface 208, input device 210, and/or output module 212.

Memory 202 includes any non-transitory computer-readable storage media for storing data and/or software that is executable by processor 204 and which controls the operation of workstation 80. In an embodiment, memory 202 may include one or more solid-state storage devices such as flash memory chips. Alternatively or in addition to the one or more solid-state storage devices, memory 202 may include one or more mass storage devices connected to the processor 204 through a mass storage controller (not shown) and a communications bus (not shown). Although the description of computer-readable media contained herein refers to a solid-state storage, it should be appreciated by those skilled in the art that computer-readable storage media can be any available media that can be accessed by the processor 204. That is, computer readable storage media includes non-transitory, volatile and non-volatile, removable and non-removable media implemented in any method or technology for storage of information such as computer-readable instructions, data structures, program modules or other data. For example, computer-readable storage media includes RAM, ROM, EPROM, EEPROM, flash memory or other solid state memory technology, CD-ROM, DVD, Blu-Ray or other optical storage, magnetic cassettes, magnetic tape, magnetic disk storage or other magnetic storage devices, or any other medium which can be used to store the desired information and which can be accessed by workstation 80.

Memory 202 may store application 81 and/or CT data 214. Application 81 may, when executed by processor 204, cause display 206 to present user interface 216. Network interface 208 may be configured to connect to a network such as a local area network (LAN) consisting of a wired network and/or a wireless network, a wide area network (WAN), a wireless mobile network, a Bluetooth network, and/or the internet. Input device 210 may be any device by means of which a user may interact with workstation 80, such as, for example, a mouse, keyboard, foot pedal, touch screen, and/or voice interface. Output module 212 may include any connectivity port or bus, such as, for example, parallel ports, serial ports, universal serial busses (USB), or any other similar connectivity port known to those skilled in the art.

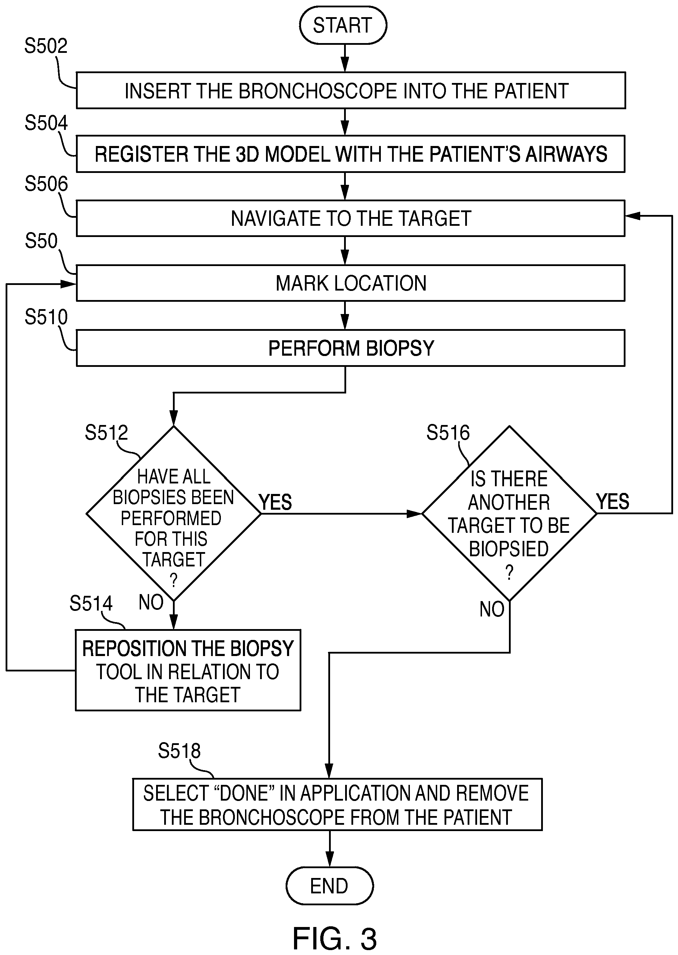

Referring now to FIG. 3, there is shown a flowchart of an example method for digitally marking the location where a tissue sample is obtained during a biopsy procedure. Prior to the start of navigation, the clinician loads a navigation plan into application 81 from memory 202, a USB device, or from network interface 208. Initially, LG 92 and EWC 96 are locked together via locking mechanism 99 and inserted into bronchoscope 50 such that EM sensor 94 projects from the distal end of bronchoscope 50. The clinician then inserts bronchoscope 50 into the patient in step S502. Bronchoscope 50 may, for example, be inserted via the patient's mouth or nose. Alternatively, EM sensor 94 may be embedded within the distal tip of EWC 96 and may operate independently of LG 92.

The clinician advances bronchoscope 50, LG 92, and EWC 96 into each region of the patient's airways in step S504 until registration has occurred between the location of EM sensor 94 of LG 92 and the 3D volume of the navigation plan. Further disclosure of the process of registration is disclosed in U.S. Patent Application No. 62/020,220, entitled REAL-TIME AUTOMATIC REGISTRATION FEEDBACK, filed on July 2, 2014.

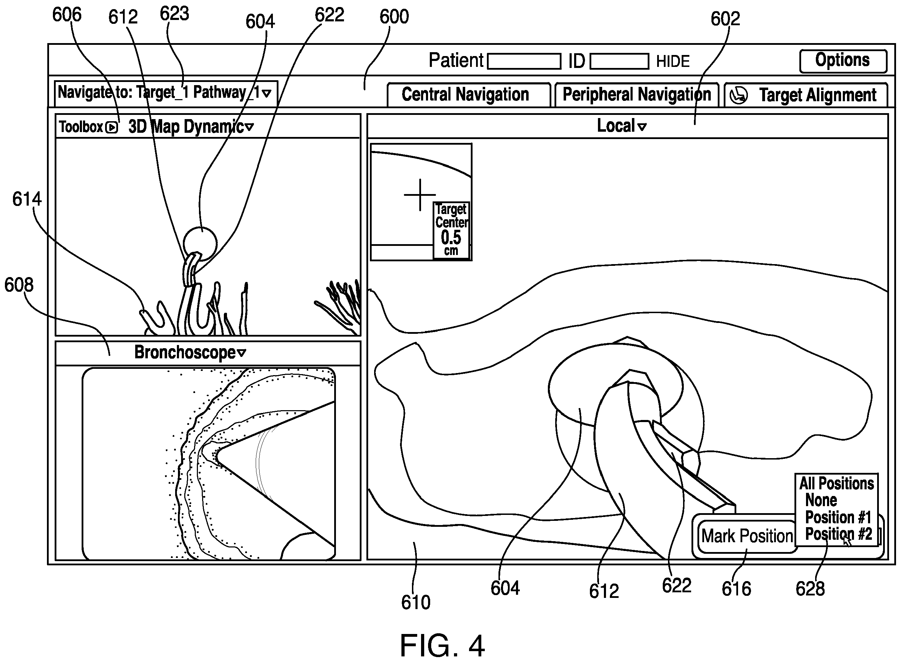

Once registration is complete, user interface 216 presents the clinician with a view 600 as shown in FIG. 4 to assist the clinician in navigating LG 92 and EWC 96 to the target 604. View 600 may include a local view 602, a 3D map dynamic view 606, and a bronchoscope view 608. Local view 602 presents the clinician with a slice 610 of the 3D volume located at and aligned with the distal tip 93 of LG 92. The slice 610 is presented from an elevated perspective. Local view 602 also presents the clinician with a visualization of the distal tip 93 of LG 92 in the form of a virtual probe 612. Virtual probe 612 provides the clinician with an indication of the direction that distal tip 93 of LG 92 is facing so that the clinician can control the advancement of the LG 92 and EWC 96 in the patient's airways.

3D map dynamic view 606 presents a dynamic 3D model 614 of the patient's airways generated from the 3D volume of the loaded navigation plan. The orientation of dynamic 3D model 614 automatically updates based on movement of the EM sensor 94 within the patient's airways to provide the clinician with a view of the dynamic 3D model 614 that is relatively unobstructed by airway branches that are not on the pathway to the target 604. 3D map dynamic view 606 also presents the virtual probe 612 to the clinician as described above where the virtual probe 612 rotates and moves through the airways presented in the dynamic 3D model 606 as the clinician advances the EM sensor 94 through corresponding patient airways.

Bronchoscope view 608 presents the clinician with a real-time image received from the bronchoscope 50 and allows the clinician to visually observe the patient's airways in real-time as bronchoscope 50 is navigated through the patient's airways toward target 604.

The clinician navigates bronchoscope 50 toward the target 604 until the patient's airways become too small for bronchoscope 50 to pass and wedges bronchoscope 50 in place. LG 92 and EWC 96 are then extended from bronchoscope 50 and the clinician navigates LG 92 and EWC 96 toward the target 604 using view 600 of user interface 216 until virtual probe 612 is adjacent to or inserted into target 604, as shown, for example, in FIG. 4.

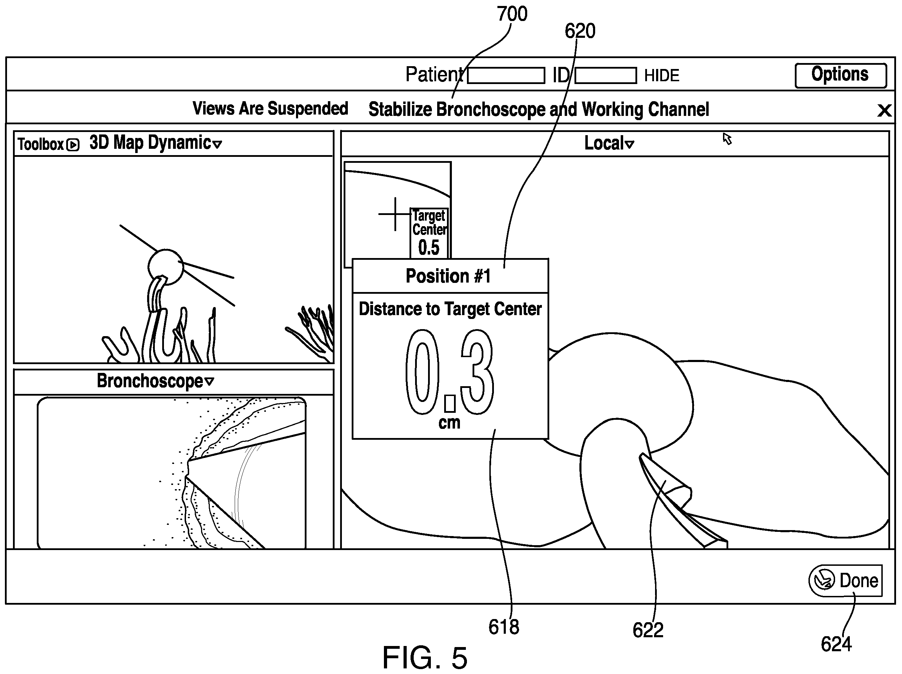

The clinician then begins the biopsy by activating a "mark position" button 614 to virtually mark the position of virtual probe 612 in the 3D volume which corresponds to the registered position of EM sensor 94 in step S508. Activating the "mark position" button 616 causes user interface 216 to present a view 700 including details of the marked position, as shown in FIG. 5. For example, view 700 may indicate a distance to the target center 618 and a biopsy position number 620.

After activating the "mark position" button 616, the clinician may remove LG 92 from EWC 96 and bronchoscope 50 and insert a biopsy tool 102 into bronchoscope 50 and EWC 96 to obtain a tissue sample at the target 604 in step S510. In some embodiments, the clinician then removes biopsy tool 102 from EWC 96 and bronchoscope 50 and reinserts LG 92. When LG 92 reaches the distal end of EWC 96, the clinician activates a "done" button 624 in view 700 indicating that the biopsy is complete. Though described herein in a specific order, the perform biopsy step S510 and the mark location step S508 may be performed in any order.

During the biopsy, application 81 stores the position marked by virtual probe 612 within the patient's airways and places a virtual marker 622 in both the 3D model 614 and local view 602 of view 600 to mark the location where the tissue sample was obtained. The storing of the position and placement of virtual marker 622 may be performed upon activation of the "mark position" button 616 in view 600, during the biopsy, or upon activation of the "done" button 624 in view 700. Additionally, the location where the tissue sample is obtained may also be physically marked by, for example, implanting an echogenic marker or a dye which can be detected in future CT scans of the patient and in some instances compared to the locations of the virtual markers 622 stored in the CT image data and/or the navigation plan. After the tissue sample is obtained and the location is marked, the clinician may remove biopsy tool 102 from bronchoscope 50 and provide the tissue sample to a rapid on-site evaluation ("ROSE") clinician for immediate testing or submit to a lab for routine testing.

The clinician determines in step S512 whether another biopsy needs to be performed at target 604. If another biopsy needs to be performed, the clinician repositions LG 92 relative to target 604 in step S514 using view 600 and repeats steps S508 to S512. If no further biopsies are required for target 604, the clinician determines if there is another target to be biopsied in step S516. For example, the clinician may activate a target selection button 623 of view 600 to see if navigation to another target has been planned. If another target is available, the clinician may activate navigation to the new target by activating target selection button 623 and may repeat steps S506 to S516 for the new target as described above.

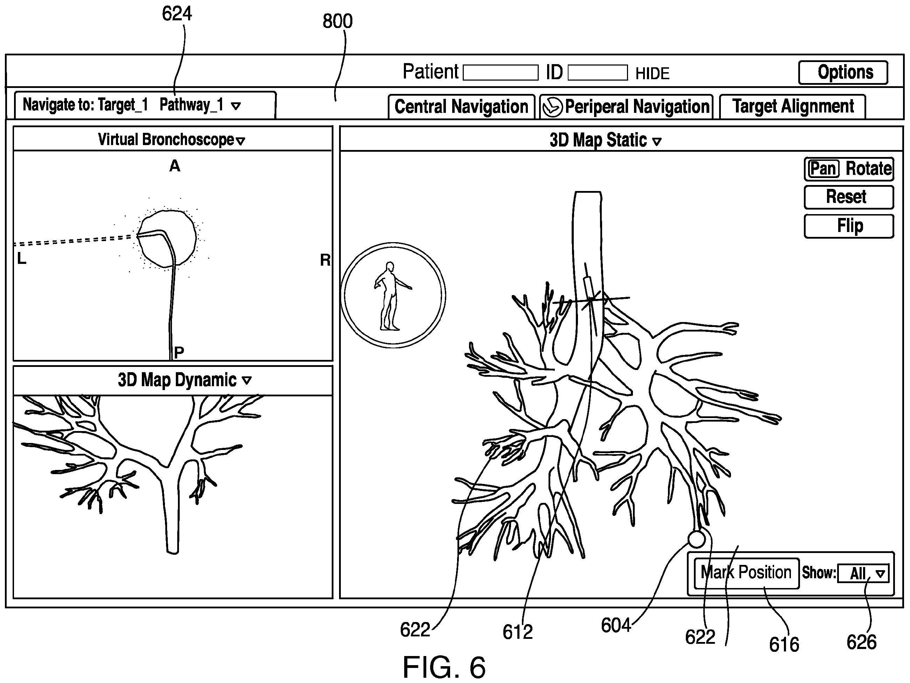

As illustrated in FIG. 6, a virtual marker 622 may presented in view 800 for each marked biopsy location and the clinician may return to a specified biopsy location at a later time, for example, upon receiving a result of the ROSE testing to perform further biopsies or treatment. The virtual marker 622 may be saved as part of the navigation plan, and may include additional information relating to the biopsy, such as the date and time when the tissue sample was obtained, the results of related testing performed on the tissue sample, and/or other information related to the biopsy. The virtual marker 622 may also be used as a future target for planning additional pathways using the navigation plan. For example, application 81 may automatically create a pathway to stored virtual markers 622 based on the pathway planned for target 604 since the pathway is already known. Alternatively, the actual path taken to the virtual marker 622 by the LG 92 may be stored in association with the virtual marker 622. The clinician may also select which virtual markers 622 are displayed by activating a virtual marker menu 626 and selecting a virtual marker position 628 corresponding to the biopsy position number 620 from view 616, as shown, for example, in FIG. 4.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting but merely as exemplifications of particular embodiments. Those skilled in the art will envision other modifications within the scope of the claims appended hereto.

## Claims

1. A system for marking a biopsy location in a representation of a patient's airways, the system comprising:
a navigation system (10) including a navigation plan which includes a CT volume generated from a plurality of CT images;
a probe (92) for insertion into a patient's airways, the probe including a location sensor (94) in operative communication with the navigation system; and
the system configured to carry out the steps of
registering a sensed location of the probe with the CT volume of the navigation plan;
selecting or enabling the selection of a target in the navigation plan;
storing a position of the location sensor in the navigation system when at the target as a biopsy location; and said system being **characterized in that** it further comprises the step of
presenting a view (602) of a 3D volume which corresponds to the registered location of the sensor (94), the view including a virtual probe (612) which provides a visualization of the distal tip (93) of the probe (94) and indicates the direction that the distal tip (93) of the probe (92) is facing.

2. The system according to claim 1, wherein the system is further configured to place a virtual marker corresponding to the biopsy location in at least one of a 3D model of the patient's airways generated from the CT volume or a local view of the patient's airways generated from a slice of the CT volume.

3. The system according to claim 1, wherein the system is further configured to store a second position of the location sensor in the navigation system as a second biopsy location.

4. The system according to claim 1, wherein the system is further configured to carry out the steps of:
selecting a second target in the navigation plan;
storing a second position of the location sensor when at the target in the navigation system as a second biopsy location.

5. The system according to claim 4, wherein the system is further configured to carry out the steps of:
reselecting at least one of the biopsy location or the second biopsy location;
presenting a pathway to the reselected at least one of the biopsy location or the second biopsy location as a return target in the navigation plan; and
storing a return position of the location sensor when at the reselected target in the navigation system as a return biopsy location.

6. The system according to claim 1, wherein the system is further configured to store a distance to a center of the target and a biopsy position number with the biopsy location.

## Patentansprüche

1. System zum Markieren einer Biopsieposition in einer Darstellung der Atemwege eines Patienten, das System umfassend:
ein Navigationssystem (10) mit einem Navigationsplan, der ein aus mehreren CT-Bildern erzeugtes CT-Volumen enthält;
eine Sonde (92) zum Einführen in die Atemwege eines Patienten, wobei die Sonde einen Positionssensor (94) in wirksamer Kommunikation mit dem Navigationssystem aufweist; und
das System, das konfiguriert ist, um die folgenden Schritte auszuführen:
Registrieren einer erfassten Position der Sonde mit dem CT-Volumen des Navigationsplans;
Auswählen oder Aktivieren der Auswahl eines Ziels im Navigationsplan;
Speichern einer Position des Positionssensors im Navigationssystem am Ziel als Biopsieposition; und
wobei das System **dadurch gekennzeichnet ist, dass** es ferner den Schritt des Darstellens einer Ansicht (602) eines 3D-Volumens umfasst, das der registrierten Position des Sensors (94) entspricht, wobei die Ansicht eine virtuelle Sonde (612) umfasst, die eine Visualisierung der distalen Spitze (93) der Sonde (94) bereitstellt und die Richtung angibt, in die die distale Spitze (93) der Sonde (92) gerichtet ist.

2. System nach Anspruch 1, wobei das System ferner so konfiguriert ist, dass es einen virtuellen Marker entsprechend der Biopsieposition in mindestens einem 3D-Modell der Atemwege des Patienten, das aus dem CT-Volumen erzeugt wird, oder einer lokalen Ansicht der Atemwege des Patienten, die aus einem Schnitt des CT-Volumens erzeugt wird, platziert.

3. System nach Anspruch 1, wobei das System ferner so konfiguriert ist, dass es eine zweite Position des Positionssensors im Navigationssystem als zweite Biopsieposition speichert.

4. System nach Anspruch 1, wobei das System ferner so konfiguriert ist, dass es die folgenden Schritte ausführt:
Auswählen eines zweiten Ziels im Navigationsplan;
Speichern einer zweiten Position des Positionssensors am Ziel im Navigationssystem als zweite Biopsieposition.

5. System nach Anspruch 4, wobei das System ferner so konfiguriert ist, dass es die folgenden Schritte ausführt:
erneutes Auswählen von mindestens der Biopsieposition oder der zweiten Biopsieposition;
Darstellen eines Pfades zu mindestens der erneut ausgewählten Biopsieposition oder der zweiten Biopsieposition als Rückkehrziel im Navigationsplan; sowie
Speichern einer Rückkehrposition des Positionssensors, wenn er sich am erneut ausgewählten Ziel im Navigationssystem befindet, als Biopsie-Rückkehrposition.

6. System nach Anspruch 1, wobei das System ferner so konfiguriert ist, dass es einen Abstand zu einem Zentrum des Ziels und eine Biopsiepositionsnummer mit der Biopsieposition speichert.

## Revendications

1. Système de marquage d'un emplacement de biopsie dans une représentation des voies respiratoires d'un patient, le système comprenant :
un système de navigation (10) comportant un plan de navigation qui comporte un volume de CT généré à partir d'une pluralité d'images de CT ;
une sonde (92) à insérer dans les voies respiratoires d'un patient, la sonde comportant un capteur d'emplacement (94) en communication opérationnelle avec le système de navigation ; et
le système configuré pour réaliser les étapes
d'enregistrement d'un emplacement détecté de la sonde avec le volume de CT du plan de navigation ;
de sélection ou de possibilité de sélectionner une cible dans le plan de navigation ;
de stockage d'un emplacement du capteur d'emplacement dans le système de navigation lorsqu'il est au niveau de la cible en tant qu'emplacement de biopsie ; et
ledit système étant **caractérisé en ce qu'**il comprend en outre l'étape de
présentation d'une vue (602) d'un volume 3D qui correspond à l'emplacement enregistré du capteur (94), la vue comportant une sonde virtuelle (612) qui fournit une visualisation de la pointe distale (93) de la sonde (94) et indique la direction dans laquelle la pointe distale (93) de la sonde (92) est orientée.

2. Système selon la revendication 1, dans lequel le système est en outre configuré pour placer un marqueur virtuel correspondant à l'emplacement de biopsie dans un modèle 3D des voies respiratoires du patient généré à partir du volume de CT et/ou une vue locale du patient des voies respiratoires du patient générée à partir d'une tranche du volume de CT.

3. Système selon la revendication 1, dans lequel le système est en outre configuré pour stocker un second emplacement du capteur d'emplacement dans le système de navigation en tant que second emplacement de biopsie.

4. Système selon la revendication 1, dans lequel le système est en outre configuré pour réaliser les étapes consistant à :
sélectionner une seconde cible dans le plan de navigation ;
stocker un second emplacement du capteur d'emplacement lorsqu'il est au niveau de la cible dans le système de navigation en tant que second emplacement de biopsie.

5. Système selon la revendication 4, dans lequel le système est en outre configuré pour réaliser les étapes consistant à :
resélectionner l'emplacement de biopsie et/ou le second emplacement de biopsie ;
présenter un chemin vers l'emplacement de biopsie et/ou le second emplacement de biopsie resélectionné(s) en tant que cible de retour dans le plan de navigation ; et
stocker une position de retour du capteur d'emplacement lorsqu'il est au niveau de la cible resélectionnée dans le système de navigation en tant qu'emplacement de biopsie de retour.

6. Système selon la revendication 1, dans lequel le système est en outre configuré pour stocker une distance vers un centre de la cible et un numéro de position de biopsie avec l'emplacement de biopsie.
